# EUROPEAN PATENT APPLICATION

(11) **EP 4 826 383 A1**
(43) Date of publication of application: **21.10.2026**
(21) Application number: 25170710.5
(22) Date of filing: 15.04.2025
(51) Int. Cl.: G01N 33/543, G01N 33/58

(54) **BREAKABLE NANOPARTICLES AND THEIR USE IN AN ELECTROCHEMILUMINESCENCE (ECL) REACTION, MORE PARTICULARLY IN AN ECL-BASED DETECTION METHOD**

(71) Applicant: UNIVERSITA' DEGLI STUDI DI MILANO, 20122 Milano (IT); Alma Mater Studiorum - Università di Bologna, 40126 Bologna (BO) (IT)
(72) Inventor: DE COLA, Luisa, 20122 Milano (IT); RIMOLDI, Isabella Silvia, 20122 Milano (IT); FACCHETTI, Giorgio, 10122 Milano (IT); ARNAL VALLÉS, Lorenzo, 10122 Milano (IT); MENDUTI, Luigi, 10122 Milano (IT); POTENTI, Simone, 10122 Milano (IT); VALENTI, Giovanni, 40126 Bologna (IT); PRODI, Luca, 40126 Bologna (IT); RAMPAZZO, Enrico, 40126 Bologna (IT)
(74) Representative: Robba, Pierpaolo

(57) **Abstract**

The present invention relates to nanoparticles including in their structure one or more cleavable bonds, that can be broken upon application of a given stimulus, thus resulting in a disintegration of the nanoparticle structure. The present invention also relates to the use of such breakable nanoparticles as platforms for luminophore units in ECL reactions. By adopting breakable nanoparticles as platforms for the emitting units, the luminophore units are no longer constrained close to one another, neglecting any quenching effect; in addition, the freely diffusing luminophore units activate the ECL catalytic pathway (homogeneous mechanism) that results very efficient for the analytical signal generation. As a result, a signal amplification that is proportional to the increase in the number of emitting units used can be aimed. The present invention may advantageously be applied to the detection of pathogens, bacteria, viruses, parasites, RNA, DNA, biomolecules and small analytes in biological samples.

## Description

### Technical field of the invention

The present invention relates to new breakable nanoparticles, which are particularly suitable to be used in electrochemiluminescence (ECL) reactions.

More particularly, the present invention relates to new breakable nanoparticles and their use in ECL reactions in the frame of ECL-detection methods.

The present invention may advantageously be applied to the detection of pathogens, bacteria, viruses, parasites, RNA, DNA, biomolecules, biomarkers, and small analytes through ECL-based detection systems.

More in detail, the present invention allows to strikingly increase the sensitivity of ECL-based detection methods.

### Prior art

Electrochemiluminescence or electrogenerated chemiluminescence (ECL) is a kind of luminescence produced during electrochemical reactions in solutions.

In ECL process, electrochemically generated species undergo high-energy electron transfer reaction to form species at an excited state that then emit light upon relaxation to a lower-level state. The wavelength of the emitted photon of light corresponds to the energy gap between these two states.

Since in ECL mechanism the light emission is triggered by an electrochemical reaction, it does not suffer from the background signal originated by the scattered light or sample autofluorescence that is present in photoluminescence-based - and, more particularly, in fluorescence-based - optical assays.

The absence of light excitation also simplifies the design and fabrication of portable, low-cost devices.

In addition, the reaction can be easily controlled by adjusting the potential applied at the electrode and enhanced selectivity of ECL analysis can be reached by varying the electrode potential.

As a result, ECL has proved to be a powerful analytical technique and is widely used in several technical fields, including immunoassay, food and water testing, biowarfare agent detection and so on.

In case of detection of an analyte from a biological sample, ECL is usually carried out in an aqueous medium and involves the provision of a luminescent species (luminophore) and a co-reactant.

Typical ECL immunoassays exploit ruthenium- or iridium-based complexes as luminophores and tri-n-propylamine (TPrA) as co-reactant.

Examples of suitable ruthenium-based and/or iridium-based complexes can be found, for instance, in documents WO 2011/067401, WO 2012/107419 and WO2014/019708.

Silica nanoparticles were synthetized for the first time by Stöber et al. in 1968 and, since then, they have generated enormous interest in multiple fields due to their high surface area, tunable porous structure and biocompatibility. In addition, silica nanoparticles can also incorporate some organic/inorganic groups in their structure to make them sensitive to certain stimuli (such as pH, light and redox species), leading to the possibility of charging such nanoparticles with desired compounds and then obtaining, upon application of the appropriate stimulus, a controlled release of such compounds.

It is known from the art the use of silica nanoparticles in ECL-based detection methods, the silica nanoparticles acting as platforms for the luminophore units and the Ru-based or Ir-based complexes being anchored to the nanoparticles surface as functional groups.

In this respect, see for instance WO 2018/037060.

In order to enhance the signal-to-noise ratio of ECL-based biosensors, it is known to replace a single [Ru(bpy)₃]²⁺ unit or similar unit based on another suitable metal complex with multinuclear complexes or polymeric systems embedding multiple identical emitting units. However, the enhancement in ECL sensitivity arising from such approach is not directly proportional to the increase in the number of emitting units used.

This is allegedly due also to the quenching effect exerted on the luminophores in the excited state by the luminophores at ground state, (or in the oxidized or reduced form) in their proximity, which also limits the possibility of further increasing the number of emitting units employed.

An attempt to reduce the quenching effect and enhance the ECL sensitivity has been made by encapsulating the luminophores within the silica nanoparticles (in this respect, see Valenti, G.; Rampazzo, E.; Bonacchi, S.; Petrizza, L.; Marcaccio, M.; Montalti, M.; Prodi, L.; Paolucci, F. - "Variable Doping Induces Mechanism Swapping in Electrogenerated Chemiluminescence of Ru(bpy)32+ Core-Shell Silica Nanoparticles" - Journal of the American Chemical Society 2016, 138 (49), 15935-15942 and in the document WO 2022/053965).

This attempt allowed increasing the ECL intensity with higher concentrations of ruthenium-based complexes; however, the increase was not linear and eventually levelled off at the highest concentrations.

Alternative attempts to enhance the ECL performance involved the use of new emitters based on modified ligands and the use of different co-reactants.

However, although ruthenium-doped silica nanoparticles have attracted much attention due to their simple preparation, easy surface modification, and excellent biocompatibility and have been proposed for the setup of ECL biosensors for micro-RNA analysis, protein detection, immunodetection and cell detection, the majority of these known approaches have disappointed the expected results in terms of ECL signal enhancement and there is still a need for nanoparticles used as platform for luminophore and leading to a satisfying enhancement in ECL sensitivity.

In view of the above, an object of the present invention is to provide new nanoparticles suitable for being used as platforms for luminophore units in ECL reactions that allow achieving a better signal amplification and an improved sensitivity.

Accordingly, another object of the present invention is to provide new solutions for using said nanoparticles as platforms in ECL-based detection methods and systems of pathogens, bacteria, viruses, parasites, RNA, DNA, biomolecules and small analytes in biological samples, allowing to achieve a better signal amplification and an improved sensitivity. These and other objects are achieved by the nanoparticles and their use in ECL reactions as claimed in the appended claims.

### Summary of the invention

The Applicants have noticed that in the solutions as proposed till now the luminophores (i.e., the complexes based on ruthenium or other rare earth metals) were usually immobilized on the surface of microbeads or microwell plates, which resulted in that the reaction taking place in the sandwich immunoassays involved in all cases ruthenium or other metals species in a heterogenous phase.

As a result, in all cases light resulted as emitted from the solid phase surface rather than directly in solution, thus reducing the possibility of efficient interactions between the species produced by the electrochemical reaction and their derivatives with the luminophores.

The Applicants found out that, in order to reach the highest ECL efficiency, the luminophores should be released from their immobilized state to become available in solution.

Accordingly, the present invention relates to nanoparticles including in their structure one or more cleavable bonds, that can be broken upon application of a given stimulus (such as light, pH, voltage, and so on), thus resulting in a disintegration of the nanoparticle structure. More in detail, the present invention relates to a nanoparticle including a three-dimensional framework of metal-oxygen bonds, wherein at least a subset of metal atoms in the framework are connected to at least another metal atom in the framework through a linker including a cleavable bond.

By adopting breakable nanoparticles as platforms for the emitting units, two main advantages can be achieved:
- the luminophore units are no longer constrained close to one another, neglecting any quenching effect;
- the freely diffusing luminophore units activate the ECL catalytic pathway (homogeneous mechanism) that results very efficient for the analytical signal generation.

As a result, a signal amplification that is proportional to the increase in the number of emitting units used can be aimed.

According a preferred embodiment of the invention, the nanoparticles include one or more cleavable bonds that can be broken upon application of voltage, preferably a low voltage. In a particularly preferred embodiment of the invention the nanoparticles include one or more redox-sensitive bonds, i.e., bonds that are broken as the result either of oxidation or of reduction.

In this preferred embodiment, upon application of an anodic potential, the co-reactant electrogenerated reducing / oxidating radicals can cleave the bonds due to the reduction / oxidation of the bond, thus breaking the nanoparticle and releasing its fragments into the solution.

Examples of bonds that can be broken by oxidation include aliphatic peroxide or tertiary amine.

In a particularly preferred embodiment of the invention, the nanoparticles include one or more bonds that can be broken by reduction, and most preferably a disulfide bond, as the S-S moiety is easily reduced to thiols at a reduction potential less negative than - 1,5 V. Nevertheless, in alternative embodiments of the invention, cleavable bonds that can be broken upon application of a different chemical stimulus or upon application of a physical stimulus (e.g., temperature) can be envisaged.

According to preferred embodiments of the invention, the nanoparticles are silica nanoparticles. More particularly, the nanoparticles are either organosilica nanoparticles (OSCs) or mesoporous silica nanoparticles (MSNs).

According to these embodiments, at least a subset of silicon - oxygen (silica) groups in the framework of the nanoparticle are connected to at least another silica group in the framework through a linker including a cleavable bond, that can be broken upon application of a given stimulus.

Preferably, silica groups forming the structure of the nanoparticles are connected to each other through linkers including redox-sensitive bonds, that are broken as the result either of oxidation or of reduction.

Most preferably, silica groups forming the structure of the nanoparticles are connected to each other through linkers including disulfide bonds, that are broken to thiols as the result of reduction upon application of a low voltage.

The present invention also relates to functionalized nanoparticles that have the framework as described above and are grafted with one or more compounds having the general formula (I)

A - B - C (I)

wherein
A is a luminophore unit in the form of a metal complex,
B is a linker,
C is a group for anchoring to the nanoparticle.

In preferred embodiments of the invention, A is a mono-nuclear metal complex having a general formula (II) as shown below 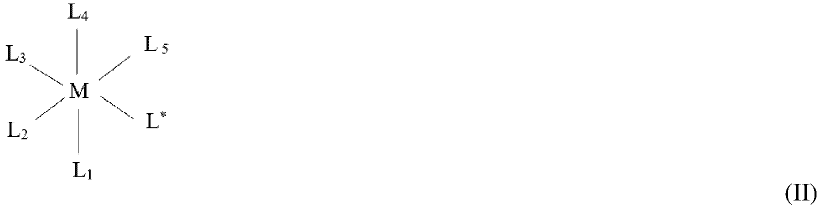 wherein
M is a metal ion
L₁ - L₅ are functional ligands bound to the central metal ion to form the metal complex L* is an ancillary ligand to which linker B is connected.

Nevertheless, in alternative embodiments of the invention, the metal complex may be a poly-nuclear metal complex, in particular a di-nuclear metal complex.

In embodiments of the invention, the metal complex comprises one or more metals selected from ruthenium (Ru(II)), rhodium (Rh(I)), osmium (Os(II)), zinc (Zn(II)), chromium (Cr(III)), platinum (Pt), palladium (Pd), iridium (Ir(III)), copper (Cu(I)), rhenium (Re(I)), aluminum (Al(III)), gallium (Ga(III)) and the rare earth metals.

In a particularly preferred embodiment of the invention, metal is selected among ruthenium and iridium. Therefore, in a particularly preferred embodiment of the invention, A is selected among ruthenium-based complexes and iridium-based complexes.

The ligands - namely the ancillary ligand - may be monodentate, bi-dentate or generally polydentate linkers. Macrocyclic, monocyclic or, optionally bridged, polycyclic moieties may also be used.

Preferred ligands are selected from substituted or unsubstituted: 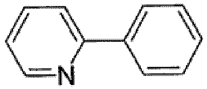 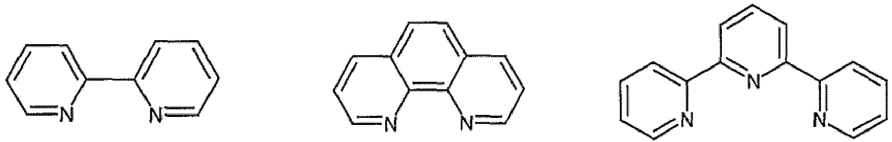 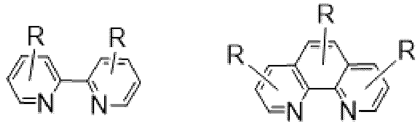 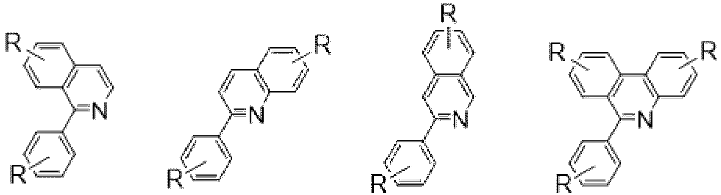
wherein R is selected from -CH₂OH, -CH₂O-(CH₂)NSO₃⁻, -CH₃, -H, -CH₂O-Si(O-(CH₂)NCH₃)₃, -(CH₂)n, -F, -(CF₂)NCF₃, -OMe, -CF₃, -NO₂, -NH₂, -SH, -CHO, -COOH and -CN.
or are defined as a moiety of the general formula (III): 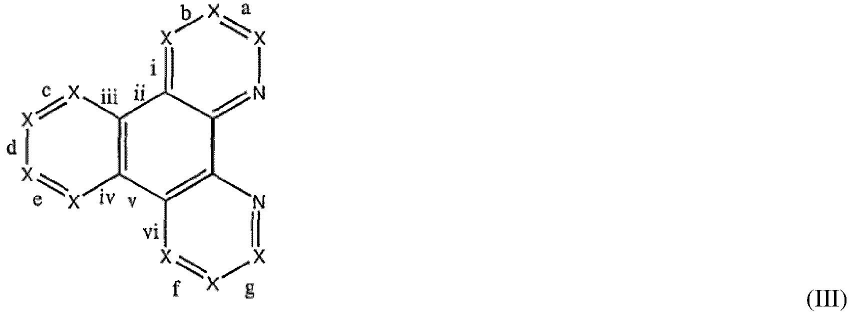
wherein X is independently CH or N, preferably at least one of the groups X being N, and the bonds a, b, c, d, e, f, and g, and the combination of bonds i/ii/iii and iv/v/vi are optionally condensed with a benzene group or a condensed aromatic moiety, wherein aromatic carbon atoms may be replaced by nitrogen atoms and wherein the complexing moiety may be substituted with C₁₋₆ alkyl, C₂₋₆ alkenyl, C₂₋₆ alkynyl, C₃₋₄ alkylene, CN, halogen, COOH, C₁₋₃ alkyl-COOH, NO₂, NH₂, or a pending group for further functionalization or complexation.

More preferred are the complexes wherein two of the groups X are N.

Macrocyclic moieties such as phthalocyanine and porphyrin moieties are also preferred. The invention may be implemented by using different linkers.

In preferred embodiments of the invention, B is an organic chain such as an aromatic chain or an aliphatic chain.

In preferred embodiments of the invention, B comprises a backbone length of between 1 and 200 atoms.

In one embodiment, the B has as a backbone a straight or branched saturated, unsaturated, unsubstituted or substituted C1-C200 alkyl chain, or a 1 to 200 atom chain consisting of carbon atoms, substituted carbon atoms and/or one or more atoms selected from O, N, P and S, or substituted N, P, S atoms, or a chain as described before with the backbone containing one or more cyclic or heterocyclic aromatic or non-aromatic ring systems.

In one embodiment, B has as a backbone a straight or branched saturated, unsaturated, unsubstituted or substituted C1-C100 alkyl chain, or a 1 to 100 atom chain consisting of carbon atoms, substituted carbon atoms and/or one or more atoms selected from O, N, P and S, or substituted N, P, or S atoms, or a chain as described before with the backbone containing one or more cyclic or heterocyclic aromatic or non-aromatic ring systems.

In one embodiment, B has as a backbone a straight or branched saturated, unsaturated, unsubstituted or substituted C1-C50 alkyl chain, or a 1 to 50 atom chain consisting of carbon atoms, substituted carbon atoms and/or one or more atoms selected from O, N, P and S, or substituted N, P, or S atoms, or a chain as described before with the backbone containing one or more cyclic or heterocyclic aromatic or non-aromatic ring systems.

In one further embodiment, B has as a backbone a straight or branched saturated, unsaturated, unsubstituted or substituted C1-C20 alkyl chain, or a 1 to 20 atom chain consisting of carbon atoms, substituted carbon atoms and/or one or more atoms selected from O, N, P and S, or substituted N, P, or S atoms, or a chain as described before with the backbone containing one or more cyclic or heterocyclic aromatic or non-aromatic ring systems.

In one embodiment, B has as a backbone a straight or branched saturated, unsaturated, unsubstituted, substituted C1-C20 alkyl chain, or a C1-C20 arylalkyl chain (wherein e.g. a phenylene ring accounts for a length of four carbon atoms), or a 1 to 20 atom chain with a backbone consisting of carbon atoms, substituted carbon atoms and/or one or more atoms selected from O, N, P and S, or substituted N, P, or S atoms, or a 1 to 20 atom chain, or with a backbone consisting of carbon atoms, substituted carbon atoms and one or more atoms selected from O, N, P and S, or substituted N, P, or S atoms comprising at least one aryl, heteroaryl, substituted aryl or substituted heteroaryl group (wherein e.g. a phenylene ring accounts for a length of four atoms).

In one embodiment, B has as a backbone a saturated C1-C12 alkyl chain, or a C1-C12 arylalkyl chain, or a 1 to 12 atom chain with a backbone consisting of carbon atoms, substituted carbon atoms and one or more atoms selected from O, N, P and S, or substituted N, P, or S atoms, or a 1 to 12 atom chain with a backbone consisting of carbon atoms, substituted carbon atoms and one or more atoms selected from O, N, P and S, or substituted N, P, or S atoms comprising at least one aryl, heteroaryl, substituted aryl or substituted heteroaryl group (wherein e.g. a phenylene ring accounts for a length of four atoms).

In one embodiment, the linker B, that includes the cleavable bond, comprises one or more amino acid(s).

In one embodiment, the linker B comprises one or more nucleotide(s).

In one embodiment, the linker B comprises a peptide chain.

In embodiments of the invention, B includes a cleavable bond that can be broken upon application of a given stimulus, such as pH, light, voltage and so on.

In a particularly preferred embodiment of the invention, B includes a redox-sensitive bond, i.e., a bond that is broken as the result either of oxidation or of reduction.

Examples of bonds that can be broken by oxidation include aliphatic peroxide or tertiary amine.

In a particularly preferred embodiment of the invention, B includes a bond that can be broken by reduction, and most preferably a disulfide bond, as the S-S moiety is easily reduced to thiols at a reduction potential less negative than -1,5 V.

Nevertheless, in alternative embodiments of the invention, cleavable bonds that can be broken upon application of a different chemical stimulus or upon application of a physical stimulus (e.g., temperature) can be envisaged.

In addition, in further alternative embodiments of the invention, B does not include any cleavable bond.

Indeed, the fragmentation of the breakable nanoparticles according to the invention is per se sufficient for obtaining freely diffusing luminophore units, that are no longer constrained close to one another, thus neglecting any quenching effect.

In a particularly preferred embodiment of the invention, C - which allows the compound including the luminophore unit to be covalently bonded to the nanoparticle - may be a silane or may include a silane.

In other preferred embodiments of the invention, C may be, for instance, amine (-NH₂), amide, azide (-N₃), carboxylate (-COOH), hydroxy (-OH), thiol (-SH), alkyne (-C≡CH), alkene (-CH=CH₂) , carbamate (-NH-CO-OR/RNH-CO-O-) , isocyanate (-NCR), trialkoxysilane group (-Si(OR)₃), carboxyl group, streptavidin or N-hydroxysuccinimide group, aldehyde, carboxylic acid, carboxylic acid ester, epoxide, N- hydroxysuccinimide ester, halogen, hydrazine, hydroxyl, sulfhydryl, maleimido, alkynyl, isothiocyanate or phosphoramidite.

According to the invention, the breakable nanoparticles can be grafted by decorating their surface with the compounds of formula (I) including the luminophore units.

In addition, according to the invention, the breakable nanoparticles can be filled within their porous structure with compounds of formula (I) including the luminophore units, the corresponding luminophore units being released after the breaking of the nanoparticles. The filling can be achieved by covalently linking the luminophores (for instance by providing a silane group as group for anchoring the compounds of formula (I) to the nanoparticle) or by embedding it by impregnation or sublimation.

The possibility of filling the breakable nanoparticles with the above-mentioned compounds allows dramatically increasing the number of used emitting units; on the other hand, such increase is not restricted by the limitations of prior solutions related to the quenching effect, as the luminophores are released into the solutions upon breaking of the cleavable bonds of the nanoparticles structure and do not interfere with one another.

In addition, the luminophores inside the pore(s) can be different from those on the surface. In general, the same nanoparticle can be used as platform for luminophore of different types and having different properties (e.g. including different metal ions, emitting light with different colors, and so on)

Theoretical models and experimental tests carried out on breakable nanoparticles with different morphologies decorated and/or filled with the above-mentioned compounds have shown that a signal amplification of several orders of magnitude greater than the signal amplification obtained in conventional ECL systems can be achieved.

Thanks to the possibility of strikingly increasing the sensitivity of the ECL system, the invention can advantageously be employed in detecting pathogens, bacteria, viruses, parasites, RNA, DNA, biomolecules, biomarkers, and small analytes in biological samples. In this respect, it shall be noted that the nanoparticles can be grafted to biomolecules (antibody, peptide nucleic acid) / anchoring groups for the detection of analytes.

It will be evident to the person skilled in the art that the embodiments described in detail in the present disclosure should in no way be understood in a limiting sense, and that numerous modifications and variants are possible without thereby departing from the scope of protection as defined by the appended claims.

### Brief description of the drawings

Theoretical models and experimental tests relating to the complexes according to embodiments of the invention are shown in the annexed drawings, in which:
- Figure 1 schematically shows the structure of a breakable silica nanoparticle according to the invention and the related disintegration process;
- Figure 2a schematically shows an example of non-breakable nanoparticle, involving a non-breakable organosilica nanoparticle (OSC), grafted with luminophore units;
- Figure 2b schematically shows a first example of nanoparticle according to the invention, involving a breakable organosilica nanoparticle (ssOSC), grafted with luminophore units;
- Figure 2c schematically shows a second example of nanoparticle according to the invention, involving a breakable mesoporous silica nanoparticle (ssMSN), grafted with luminophore units;
- Figure 3 schematically shows the theoretical model of the cleavage mechanism of the functional groups of the nanoparticles of Figure 2b;
- Figure 4 shows the results of CV-ECL measurements performed using functionalized nanoparticles as shown in Figure 2a;
- Figure 5 shows the results of CV-ECL measurements performed using functionalized nanoparticles as shown in Figure 2b;
- Figure 6 shows the results of CV-ECL measurements performed using functionalized nanoparticles as shown in Figure 2c;
- Figure 7 shows the ECL intensity profiles resulting from CV-ECL measurements performed using functionalized nanoparticles as shown in Figure 2a, Figure 2b and Figure 2c with a first concentration of co-reactant;
- Figure 8 shows the ECL intensity profiles resulting from CV-ECL measurements performed using functionalized nanoparticles as shown in Figure 2a, Figure 2b and Figure 2c with a second, different concentration of co-reactant;
- Figure 9 shows the derived count rate as a function of time obtained by using functionalized nanoparticles as shown in Figure 2b and Figure 2c.

### Description of embodiments

In the following description of embodiments of the invention reference will be made to silica nanoparticles.

However, these embodiments shall not be in no way intended as limiting the scope of protection of the invention and other nanoparticles can be used as well.

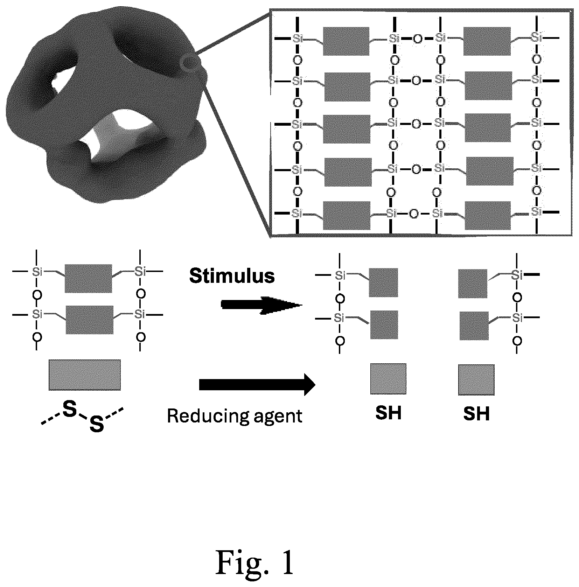

With reference to Figure 1, the present invention relates to a nanoparticle including a three-dimensional framework of metal-oxygen bonds, namely of Si-O bonds wherein at least a subset of Si atoms in the framework are connected to at least another Si atom in the framework through a linker including a cleavable bond.

In the shown embodiment, the cleavable bond is a redox-sensitive bond.

More particularly, the cleavable bond is a disulfide bond, which is sensitive to reduction. Selection of disulfide bond among possible redox sensitive bonds may represent a considerable advantage, as the S-S moiety is easily reduced (for an applied voltage less negative than -1,5 V) to thiols, according to the reaction below:

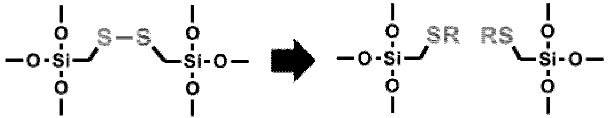

As a result, upon application of a suitable anodic potential, the reactivity of the disulfide bond is "switched on" by the co-reactant electrogenerated reducing radicals that cleave the disulfide bond due to the reduction of the S-S moiety to thiols, thus releasing the generated nanoparticle fragments into the solution.

In case the nanoparticle is grafted with functional groups, the functional groups anchored to the nanoparticles fragments are correspondingly released into the solution and made free to diffuse in said solution.

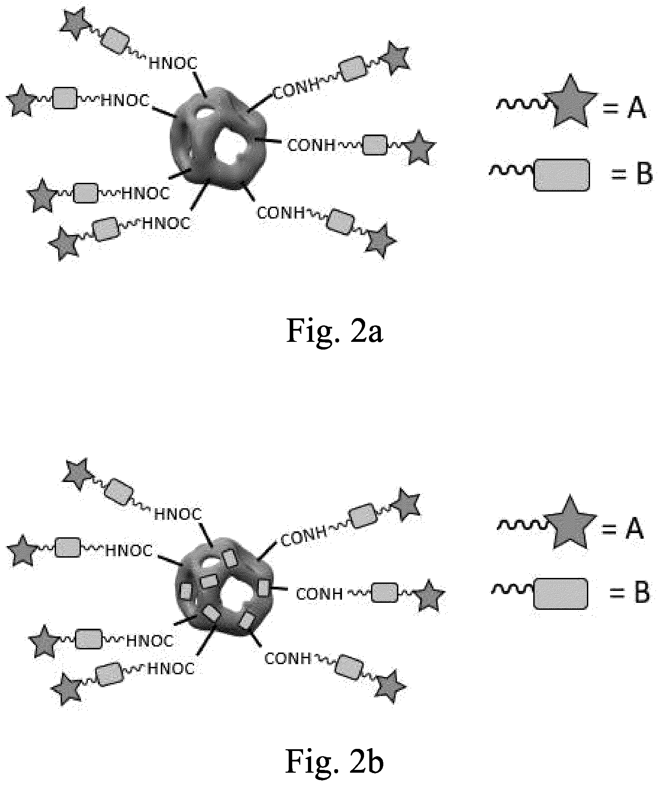

Referring to Figure 2a - 2c, three examples of nanoparticles grafted with luminophore units are schematically shown.

Specifically referring at first to Figure 2a, a non-breakable organosilica nanoparticle (OSC), which do not form part of the present invention, is shown.

This nanoparticle is grafted with a plurality of compounds having the general formula (I) as functional groups.

Since the nanoparticle of Figure 2a is a non-breakable nanoparticle, in order to avoid quenching effect, an emitting unit including a cleavable bond shall be used.

More specifically, in the example shown in Figure 2a:
A is a ruthenium-based complex, namely tris(2,2'-bipyridine)ruthenium(II) ([Ru(bpy)₃]²⁺);
B is an organic chain including a disulfide moiety; and
C is an amide group.

The surface of the nanoparticle (or nanocage) is decorated with the above-mentioned functional groups.

In the example shown in Figure 2b, a breakable organosilica nanoparticle (ssOSC) according to the invention is shown.

As in the previous example, this nanoparticle is grafted with a plurality of compounds having the general formula (I) as functional groups.

More specifically, in the example shown in Figure 2b:
A is a ruthenium-based complex, namely tris(2,2'-bipyridine)ruthenium(II) ([Ru(bpy)₃]²⁺);
B is an organic chain including a disulfide moiety; and
C is an amide group.

However, it should be noted that - thanks to the provision of cleavable bond in the structure of the nanoparticles itself - conventional luminophore units without any cleavable bond could also be used.

The surface of the nanoparticle (or nanocage) is decorated with the above-mentioned functional groups.

In addition, the porous structure of the nanocage is filled with additional compounds having the general formula (I) as functional groups.

The possibility of filling the nanocage with further luminophores, in addition to those decorating the surface of said nanocage, allows strikingly increasing the rate of luminophores per nanoparticle.

In this case, too, the porous structure of the nanocage could be filled either with luminophore units with a cleavable bond or with conventional luminophore units without any cleavable bond. In both cases, the quenching effect can be avoided thanks to breakable structure of the nanoparticle itself.

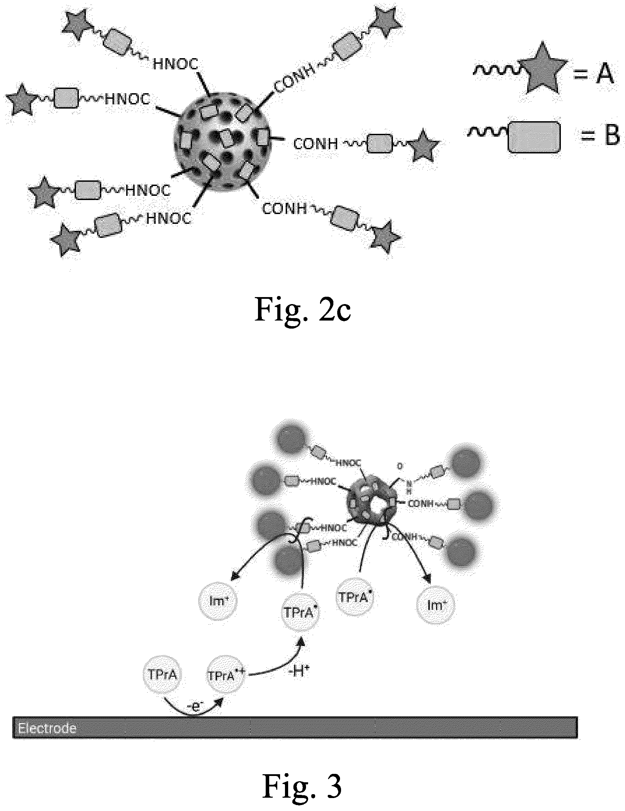

Finally, in the example shown in Figure 2c, a breakable mesoporous nanoparticle (ssMSN) according to the invention is shown.

As in the previous examples, this nanoparticle is grafted with a plurality of compounds having the general formula (I) as functional groups.

More specifically, in the example shown in Figure 2c:
A is a ruthenium-based complex, namely tris(2,2'-bipyridine)ruthenium(II) ([Ru(bpy)₃]²⁺);
B is an organic chain including a disulfide moiety; and
C is an amide group.

However, it should be noted that - thanks to the provision of cleavable bond in the structure of the nanoparticles itself - conventional luminophore units without any cleavable bond could also be used.

The surface of the nanoparticle (or nanocage) is decorated with the above-mentioned functional groups.

In addition, the porous structure of the nanocage is filled with additional compounds having general formula (I) as functional groups.

Therefore, in this example, too, the possibility of filling the nanocage with further luminophores, in addition to those decorating the surface of said nanocage, allows strikingly increasing the rate of luminophores per nanoparticle.

In this case, too, the porous structure of the nanocage could be filled either with luminophore units with a cleavable bond or with conventional luminophore units without any cleavable bond. In both cases, the quenching effect can be avoided thanks to breakable structure of the nanoparticle itself.

The model of the mechanism of the ECL reaction that can be obtained by using the nanoparticles according to the invention is schematically shown in Figure 3.

By way of non-limiting examples, Figure 3 makes reference to an ssOSC nanocage as shown in Figure 2b.

In Figure 3, tri-n-propylamine (TPrA) is used as co-reactant.

The functional groups carrying the luminophores are anchored to the nanocage by the terminal amide moiety available for conjugation in a resting state, without any electrochemical stimulus.

Upon application of a suitable anodic potential, TPrA is oxidized to TPrA^{•+} (starting at 0.83 V vs Ag/AgCl) and promptly deprotonates into TPrA^{•}. The co-reactant electrogenerated radicals are strongly reducing and reduce the cleavable bonds of the nanoparticles (and in this case of the functional groups as well).

In this respect, it is worth mentioning that selecting a disulfide bond as a cleavable bond is very advantageous, since the S-S moiety reduces to thiols at very low potentials (less negative than -1,5 V).

Upon cleavage of the disulfide bonds, the structure of the nanoparticle is broken and the luminophore units are free in the solution, thereby activating the ECL catalytic mechanism, where the oxidized Ru(II) homogeneously oxidizes TPrA.

In the example shown in Figure 3, both the disulfide moieties of the functional groups decorating the surface of the nanocage and the disulfide moieties of the functional groups inserted in the porous structure of the nanocage are cleaved by the co-reactant electrogenerated reducing radicals, so that a high number of luminophore units can be released into the solution.

Since the luminophore units are no longer immobilized in the nanocage or at the nanocage surface, they do not interfere with one another and the quenching effect can be avoided.

### Experimental tests and results

### CV-ECL measurements on OSC nanocages

CV (cyclic voltammetry)-ECL measurements were performed in a quartz cuvette containing equimolar solutions of Ru(II)-grafted OSC nanocages as shown in Figure 2a, namely 19.9 nM in a 0.3 M phosphate buffer (PB) solution at pH 6.8 supplemented with 20 mM TPrA as ECL co-reactant. The concentration of Ru(II) in solution was 8.75 µM. The working electrode potential was scanned at 100 mV/s from OCP up to 2.5 V (vs Ag/AgCl), back to 0 V (vs Ag/AgCl) and, eventually, terminating the cycle at OCP. For measuring the current intensity, a picoammeter was set at the 000.0 µA amplification level for the detection of the ECL signal.

The experimental setup consisted of a three-electrode configuration including a 3 mm glassy carbon (GC) working electrode, an Ag/AgCl (Sat'd KCl) reference electrode and a Pt wire counter electrode.

The ECL signal emitted by Ru(II) labels was recorded with a photomultiplier tube (PMT) set at a constant amplification level for all the measurements.

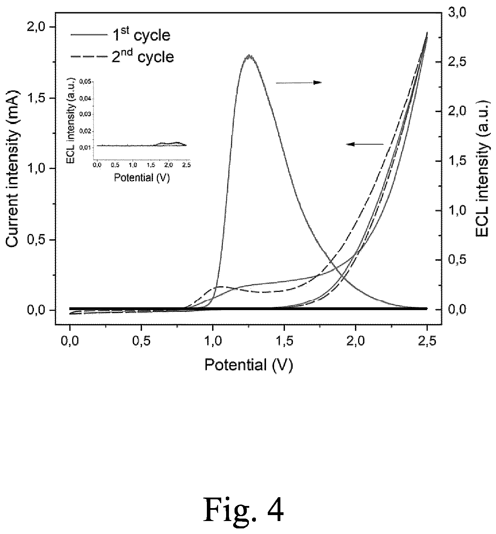

The results of the CV-ECL measurements during a first CV-ECL cycle are shown in Figure 4, while the results of the CV-ECL measurements during a second CV-ECL cycle are shown in the inset of Figure 4.

The first CV-ECL cycle of the OSC nanocage reveals a strong emission peak at 1.25 V vs Ag/AgCl, primarily ascribed to the initial prevalence of the heterogeneous mechanism driven by the TPrA oxidation at the electrode.

However, in the second cycle, the ECL emission intensity experiences significant attenuation by ~2 orders of magnitude. This decrease can be reasonably attributed to the passivation of the electrode surface due to the attachment of terminal thiols originating from the cleaved disulfide bond.

Notably, despite the emission intensity drop, the ECL signal remains discernible, with two distinct peaks clearly resolved. The first peak corresponds to the heterogeneous mechanism, manifesting at less positive potential, while the second peak is associated with the catalytic (homogeneous) mechanism, indicating the release of Ru(II) labels into the solution. The potential shift observed for these peaks compared to the one recorded during the first cycle is attributable to the surface passivation effect that increases the overpotential required for a given redox event to occur.

### CV-ECL measurements on ssOSC nanocages

CV-ECL measurements were performed in a quartz cuvette containing equimolar solutions of Ru(ll)-grafted OSC nanocages as shown in Figure 2b, namely 19.9 nM in a 0.3 M phosphate buffer (PB) solution at pH 6.8 supplemented with 20 mM TPrA as ECL co-reactant. The concentration of Ru(II) in solution was 6.57 µM. The working electrode potential was scanned at 100 mV/s from OCP up to 2.5 V (vs Ag/AgCl), back to 0 V (vs Ag/AgCl) and, eventually, terminating the cycle at OCP. For measuring the current intensity, a picoammeter was set at the 000.0 µA amplification level for the detection of the ECL signal.

The experimental setup consisted of a three-electrode configuration including a 3 mm glassy carbon (GC) working electrode, an Ag/AgCl (Sat'd KCl) reference electrode and a Pt wire counter electrode.

The ECL signal emitted by Ru(II) labels was recorded with a photomultiplier tube (PMT) set at a constant amplification level for all the measurements.

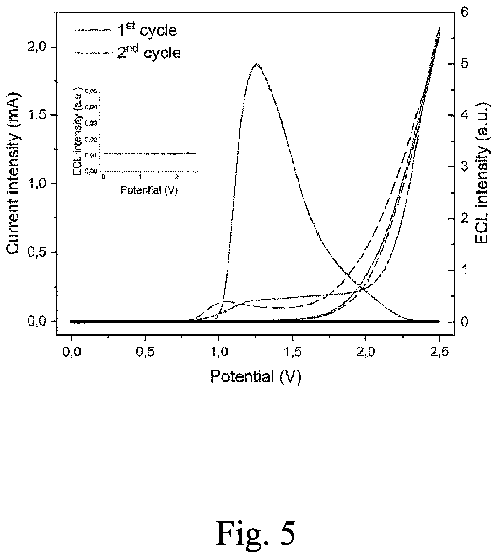

The results of the CV-ECL measurements during a first CV-ECL cycle are shown in Figure 5, while the results of the CV-ECL measurements during a second CV-ECL cycle are shown in the inset of Figure 5.

The first CV-ECL cycle of the ssOSC nanocage reveals a strong emission peak at 1.25 V vs Ag/AgCl, i.e., at the same potential as the OSC nanocage.

This suggests that likely both kinds of nanocages initially follow the same ECL generation pathway predominantly governed by the heterogeneous mechanism.

On the other hand, no ECL signal could be detected during the second CV-ECL cycle for the ssOSC sample.

This behavior could be attributed to the presence of breakable disulfide bridges on the shell of the ssOSC nanocages, which could lead to increased generation of fragments containing terminal thiols following bond cleavage during the anodic potential sweep. This enhanced thiol release could, in turn, result in a more pronounced pas sivation of the electrode, thereby impeding ECL signal generation detectable at given amplification.

### CV-ECL measurements on ssMSN nanocages

CV-ECL measurements were performed in a quartz cuvette containing equimolar solutions of Ru(ll)-grafted ssMSN nanocages as shown in Figure 2c, namely 19.9 nM in a 0.3 M phosphate buffer (PB) solution at pH 6.8 supplemented with 20 mM TPrA as ECL co-reactant. The concentration of Ru(II) in solution was 8.75 µM. The working electrode potential was scanned at 100 mV/s from OCP up to 2.5 V (vs Ag/AgCl), back to 0 V (vs Ag/AgCl) and, eventually, terminating the cycle at OCP. For measuring the current intensity, a picoammeter was set at the 000.0 µA amplification level for the detection of the ECL signal.

The experimental setup consisted of a three-electrode configuration including a 3 mm glassy carbon (GC) working electrode, an Ag/AgCl (Sat'd KCl) reference electrode and a Pt wire counter electrode.

The ECL signal emitted by Ru(II) labels was recorded with a photomultiplier tube (PMT) set at a constant amplification level for all the measurements.

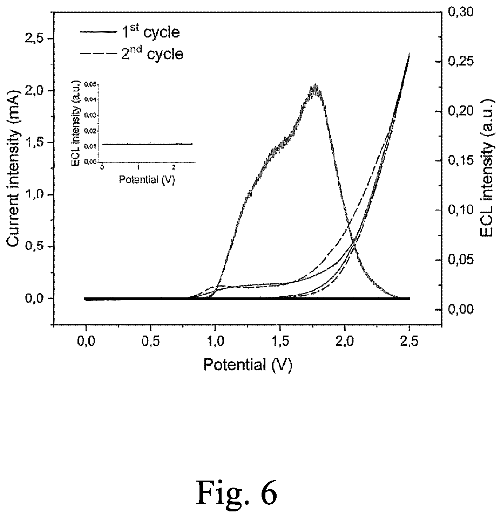

The results of the CV-ECL measurements during a first CV-ECL cycle are shown in Figure 6, while the results of the CV-ECL measurements during a second CV-ECL cycle are shown in the inset of Figure 6.

The CV-ECL curve shape for the ssMSN sample during the first cycle displays notable differences compared to those for the OSC and ssOSC samples shown in Figures 4 and 5. Specifically, a double wave pattern emerges, where the shoulder is attributed to the emission resulting from the heterogeneous mechanism, while the ECL peak at 1.8 V vs Ag/AgCl is associated with the more efficient catalytic (homogeneous) mechanism.

This discrepancy with respect to ssOSC suggests that ssMSN nanocages may undergo easier cleavage by TPrA^{•}, leading to early release of Ru(II) labels during the first cycle. Furthermore, as with ssOSC nanocages, ssMSN nanocages feature disulfide moieties within their shell, facilitating the passivation of the electrode and preventing any ECL signal to be detected during the second cycle.

### CV-ECL Measurements - Comparative Study 1

The CV-ECL curves recorded for the equimolar solution of ssOSC and ssMSN breakable nanocages were compared to the CV-ECL curve obtained for a conventional arrangement in which the nanocages are not breakable and the luminophore does not include any cleavable bond.

An Ru-silane Ru(2,2'-bipyridine-6,6'-dicarboxylate)( 4-(6-(triethoxysilyl)hexyl)pyridine)₂ was used as luminophore.

CV-ECL measurements were performed on 19.9 nM solutions of each sample in 0.3 M PB at pH 6.8 with 20 mM TPrA. The ECL signal was normalized over the esteemed number of luminophores per nanocage: ~100 Ru in OSC, ~150 in ssOSC, ~160 in ssMSN, and ~1600 in non-breakable nanocages. The working electrode potential was scanned at 100 mV/s from OCP up to 2.5 V (vs Ag/AgCl), back to 0 V (vs Ag/AgCl) and, eventually, terminating the cycle at OCP. The picoammeter was set at the 000.0 µA amplification level for the detection of the ECL signal.

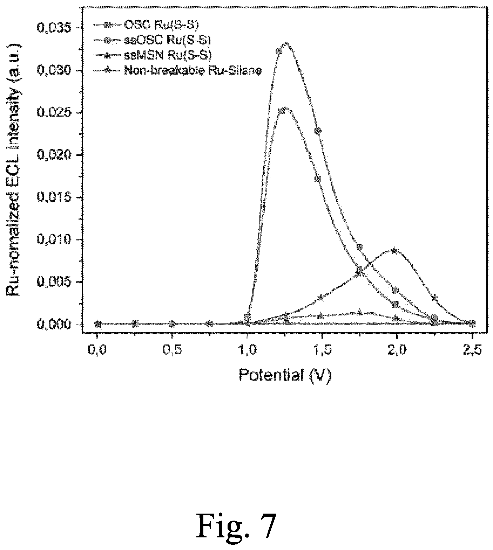

The results of the Comparative Study are shown in Figure 7.

As can be seen in Figure 7, the ssOSC exhibit stronger ECL emission than the conventional, non-breakable nanocage.

This enhanced performance can be primarily attributed to the introduction of disulfide groups within the linker connecting the nanocage and the luminophore.

Furthermore, incorporating additional disulfide bridges within the nanocage shell further improved the emission intensity as shown by the stronger ECL displayed by ssOSC nanocages compared to the OSC nanocages.

Conversely, the ssMSN nanocages generated a noticeably weaker ECL signal compared to the conventional, non-breakable nanocages.

This difference might be due to the morphology of the nanocages, which significantly affects the accessibility of TPrA radicals.

As known to the person skilled in the art, the pore dimensions play a crucial role in modulating this accessibility, potentially hindering or facilitating the ECL signal generation process.

In view of the latter assumption, the CV-ECL measurement were repeated for the ssOSC nanocages and ssMSN nanocages with a different concentration of co-reactant, namely 180 mM TprA, the other experimental conditions being unchanged.

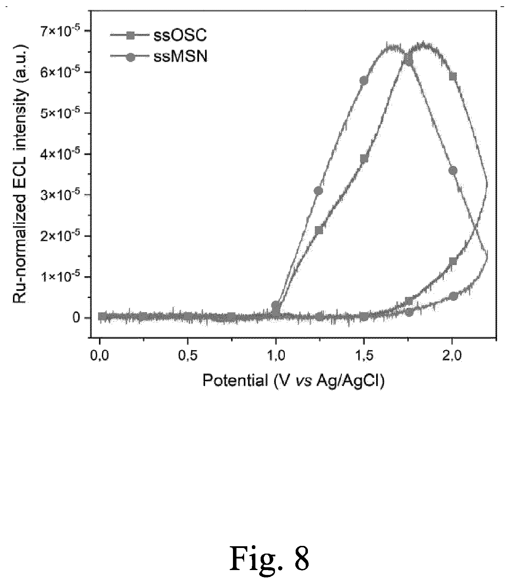

The results are shown in Figure 8.

As expected, the ECL behavior of Ru(II)-doped nanocages can be controlled by tuning the co-reactant concentration: increasing the TPrA concentration from 20 mM to 180 mM induces a change in ECL intensity ratio, making the signal of ssOSC nanocages comparable to that of ssMSN nanocages.

This can be attributed to the lower size of pores in ssMSN nanocages, which results in a lower accessibility for the TPrA radicals and in an increased difficulty in cleavage. This translates to faster cleavage of these nanocages, leading to an ECL intensity comparable to that of ssOSC nanocages.

In a nutshell, in the case of ssMSN nanocages, while a concentration of 180 mM TPrA provides sufficient radical generation to break down the particles during a CV-ECL cycle, a lower concentration of 20 mM is not sufficient.

### Rupture kinetics of breakable nanocages

The rupture kinetics of nanocages containing disulfide bridges in their shells (i.e., ssOSC nanocages and ssMSN nanocages) was investigated.

The measurements were performed using dynamic light scattering (DLS).

These measurements were performed in a 0.3 M phosphate buffer (PB) solution at pH 6.8 supplemented with 180 mM TPrA as ECL co-reactant in a plastic cuvette including a three-electrode setup: a glassy carbon working electrode (3 mm diameter), an Ag/AgCl reference electrode and a Pt wire as counter electrode. The working electrode potential was set at 1.4 V vs Ag/AgCl and it was applied for 264 straight minutes.

The derived count rate, which is the intensity of scattered light normalized by instrumental parameters, was used to monitor the rupture process over time. A decrease in the count rate indicates the breaking of nanocages, as they scatter less light as they break down.

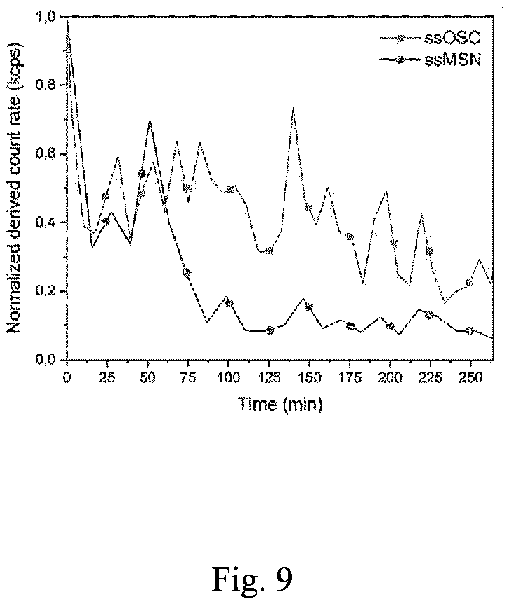

The results of this test are shown in Figure 9.

The DLS measurements reveal faster cleavage of ssMSN nanocages by TPrA^{•} radicals compared to their ssOSC counterparts.

This observation corroborates the CV-ECL experiments which showed that ssMSN undergoes cleavage in a shorter timeframe. This can be attributed to lower accessibility for TPrA radicals of ssMSN. The kinetics and fragmentation mode can be tuned by modulating the amount of disulfide groups into the silica framework from 2 to 50%.

### Grafting of luminophores (ruthenium-based complexes) on the surface of silica nanoparticles

### Silica nanocages, general procedure

A 100 mL round-bottom single-neck flask was charged with 408 mg (1.12 mmol) cetyltrimethylammonium (CTAB) and 50 mL deionized water. The mixture was magnetically stirred at 250 rpm at 50 °C for 30 min. Then, an ammonia aqueous solution (NH₃ 28-30%, 12.5 L, 0.185 mmol) was added and the stirring speed was increased to 750 rpm. Subsequently, a freshly prepared mixture (see Table 1 below for further details) of tetraethyl orthosilicate (TEOS) and bis[3-(triethoxysilyl)propyl]disulphide (BTDS) was added. The reaction mixture was stirred at 750 rpm at 50 °C for 20 h.

**Table 1**

| Type of silica nanocages | TEOS (µL) | BTDS (µL) |
|---|---|---|
| Non-breakable | 500 | 0 |
| Breakable | 448 | 103 |

### Mesoporous silica nanoparticles (MINS), general procedure

A 250 mL round-bottom single-neck flask was charged with 816 mg (2.24 mmol) cetyltrimethylammonium (CTAB) and 50 mL deionized water. The mixture was magnetically stirred at 500 rpm at 50 °C for 30 min. Then, an ammonia aqueous solution (NH₃ 28-30%, 25 L, 0.37 mmol) was added and the stirring speed was increased to 750 rpm. Subsequently, a freshly prepared mixture (see Table 2 below for further details) of tetraethyl orthosilicate (TEOS) and bis[3-(triethoxysilyl)propyl]disulphide (BTDS) was added. The reaction mixture was stirred at 750 rpm at 50 °C for 20 h.

**Table 2**

| Type of MSN | TEOS (µL) | BTDS (µL) |
|---|---|---|
| Non-breakable | 1000 | 0 |
| Breakable | 1000 | 241 |

### Grafting

A round-bottom single-neck flask was charged with a freshly prepared batch of undialyzed silica nanoparticles (either amino- or carboxylate-grafted) and phosphate buffer (0.2 M, pH 7.4, 1:1 v/v with respect to the colloidal nanoparticle suspension).

Subsequently, the suited Ru dye was added to the mixture (10-20 mol% with respect to TEOS, i.e., 1 equiv. with respect to the amino-/carboxylic-bearing grafting agent). Once the Ru dye was completely dissolved, the amide coupling system (such as EDC/NHS or TSTU, 1 equiv. with respect to TEOS) was added to the mixture, which was vigorously stirred at room temperature overnight. The particle dispersion was purified by dialysis against an EtOH/H₂O/AcOH (1:1:0.007 v/v/v) mixture for 3 days (changing the dialysis bath daily), then against an EtOH/H₂O (1:1 v/v) mixture for 1 day, and finally against H₂O for 1 day.

### Synthesis of breakable and non-breakable nanocages containing the non-breakable complex Na₂[Ru(SulfoBipy)₂(Bipy-NHS)]

Initially, the silane derivative was prepared by reacting the NHS group with the amino group of the APTES. The isolated system was then used to make the cage derivatives using the following protocol.

The chosen Ru dye (10-20 mol% with respect to TEOS, depending on the chosen nanoparticles to be grafted) was dissolved in 200 µL of anhydrous DMSO within a 2 mL Eppendorf tube equipped with a magnetic stirring bar. One equivalent of the suited silane (either 3-aminopropyl- or 3-isocyanatopropyl-silane, to be reacted with a NHS ester bearing Ru dye or with an amino-bearing Ru dye, respectively) was added to the mixture, which was vigorously stirred in the dark at room temperature for half an hour.

(If the amino-bearing Ru dye was isolated as an ammonium salt, remember to add 1 equiv. of a suited organic base to generate the free amine.)

The silanized Ru dye was not isolated: the whole mixture was added to the nanoparticle colloidal suspension, as reported in square brackets in the general protocols for the synthesis of silica nanoparticles. The identity of the silanized Ru dye can be confirmed via ESI-MS analysis.

### Synthesis of breakable and non-breakable capsules containing the non-breakable complex Na₄[Ru(SulfoBipy)₃]

To have a full encapsulated system and study a possible release from a closed nanomaterial finally the tris-sulfonated compound Na₄[Ru(SulfoBipy)₃] was encapsulated in breakable and non-breakable nanocapsules with the same standardized procedure: in a nanoemulsion synthesis in which the water soluble compound is dissolved in a minimum amount of water (see scheme below), 6 mg/mL and 30 mg/mL (final concentration) ruthenium complex water solution, Triton X-100 and n-hexanol were dissolved in cyclohexane. Separately, the aqueous solution of the complex was mixed with tetraethyl orthosilicate (TEOS) and bis[3-(triethoxysilyl)propyl]disulfide.

After mixing manually, this mixture was added to the former organic medium. Eventually, 100 µL 28 % ammonia aqueous solution was added and the water-oil emulsion was stirred for 5 hours at room temperature. After that, 20 mL pure acetone was added in order to precipitate the material that was recovered by means of centrifugation and washing six times with water.

## Claims

1. Breakable nanoparticles including a three-dimensional framework of metal-oxygen bonds, wherein at least a subset of metal atoms in the framework are connected to at least another metal atom in the framework through a linker including a cleavable bond that is breakable upon application of a given stimulus.

2. Breakable nanoparticles according to claim 1, wherein said cleavable bonds are redox-sensitive bonds, cleavable as the result either of oxidation or of reduction.

3. Breakable nanoparticles according to claim 1, wherein said cleavable bonds are disulfide bonds.

4. Breakable nanoparticles according to any of claims 1 - 3, wherein said nanoparticles are silica nanoparticles, namely organosilica nanoparticles (OSCs) or mesoporous silica nanoparticles (MSNs).

5. Breakable nanoparticles according to any of claims 1 - 4, wherein said nanoparticles are grafted with one or more functional groups having a general formula (I)
A - B - C (I)
wherein
A is a luminophore unit in the form of a mono-nuclear, di-nuclear or poly-nuclear metal complex,
B is linker,
C is a group for anchoring to the nanoparticle.

6. Breakable nanoparticles according to claim 5, wherein A is a mono-nuclear metal complex having a general formula (II) as shown below 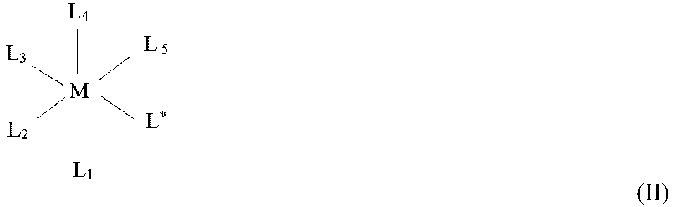 wherein
M is a metal ion
L1 - L5 are functional ligands bound to the central metal ion to form the metal complex L* is an ancillary ligand to which linker B is connected.

7. Breakable nanoparticles according to claim 5 or 6, wherein said functional groups decorate the surface of said nanoparticles.

8. Breakable nanoparticles according to claim 5 or 6 or 7, wherein said functional groups fill the porous structure of said nanoparticles.

9. Breakable nanoparticles according to any of claims 5 - 8, wherein A is selected among ruthenium-based complexes and iridium-based complexes.

10. Breakable nanoparticles according to any of claims 5 - 8, wherein B includes a cleavable bond, preferably a redox-sensitive bond, cleavable as the result either of oxidation or of reduction.

11. Use of the nanoparticles of any of claims 1 - 10 for performing an electrochemiluminescence reaction.

12. Use according to claim 11, wherein the electrochemiluminescence reaction is performed in aqueous solution.

13. Use of the nanoparticles of any of claims 1 - 10 in an electrochemiluminescence-based detection method.

14. Use according to claim 13 for detecting pathogens, bacteria, viruses, parasites, RNA, DNA, biomolecules and other small analytes in biological samples.
